Europäisches Patentamt

European Patent Office

Office européen des brevets

(19)

(11) **EP 1 046 914 B1**

(12) **EUROPEAN PATENT SPECIFICATION**

(45) Date of publication and mention
of the grant of the patent:
**02.07.2003 Bulletin 2003/27**

(51) Int Cl.⁷: **G01N 33/72**

(21) Application number: **99107880.9**

(22) Date of filing: **21.04.1999**

(54) **Method and reagents for the determination of bilirubins**

Verfahren und Reagens zur Bestimmung von Bilirubinen

Procédé et reactifs pour la détermination des Bilirubines

(84) Designated Contracting States:
**AT BE CH CY DE DK ES FI FR GB GR IE IT LI LU
MC NL PT SE**

(43) Date of publication of application:
**25.10.2000 Bulletin 2000/43**

(73) Proprietor: **Habbal, Mohammad Zouheir Dr.
Beirut (LB)**

(72) Inventor: **Habbal, Mohammad Zouheir Dr.
Beirut (LB)**

(74) Representative: **Vuillermoz, Bruno et al
Cabinet Laurent & Charras
B.P. 32
20, rue Louis Chirpaz
69134 Ecully Cedex (FR)**

(56) References cited:
**EP-A- 0 433 977        WO-A-99/04258
US-A- 4 892 833        US-A- 5 149 272**

- **DATABASE CHEMABS [Online] CHEMICAL
ABSTRACTS SERVICE, COLUMBUS, OHIO, US
FRIEDECKY, B.: "Determination of total serum
bilirubin by the N,N- dimethylformamide
reagent" retrieved from STN Database
accession no. 96:118389 XP002117331 &
BIOCHEM. CLIN. BOHEMOSLOV. (1981), 10(4),
299-302 ,**
- **CERIOTTI, G.: "Considerations on bilirubin
determination" QUAL. CONTROL CLIN. CHEM.,
TRANS. INT. SYMP., 6TH (1975), 3-10.
EDITOR(S): ANIDO, G.;VAN KAMPEN, E. J.;
ROSALKI, S. B. PUBLISHER: DE GRUYTER,
BERLIN, GER. , XP002117329**
- **MAJKIC-SINGH, NADA ET AL: "Evaluation of the
Ceriotti method for bilirubin assay" IUGOSL.
PHYSIOL. PHARMACOL. ACTA (1982), 18(3),
205-13 , XP002117330**
- **PATENT ABSTRACTS OF JAPAN vol. 009, no.
072 (P-345), 2 April 1985 (1985-04-02) & JP 59
203954 A (SHIMAZU SEISAKUSHO KK), 19
November 1984 (1984-11-19)**

EP 1 046 914 B1

**Description**

The present invention relates to a method for the determination of bilirubins in biological sera, and more particularly to a new reagent as accelerator and a new reagents composition both for use in such determination of bilirubins.

Various methods are already known for the determination of bilirubins, which rely on their reactions with diazo compounds. The diazo methods yield a single value for total bilirubin (TBR) concentration in a sample and, with certain modification, an additional smaller value is obtained which represents the concentration of the more soluble bilirubin esters or conjugates, also known as direct bilirubins (DBR). One of the known method is that of Doumas et al. (Clin. Chem. 31, 1779-1789, 1985), in which caffeine is used as an accelerator of the diazotization reaction, necessary due to the slow reaction of unconjugated bilirubin. However, caffein has disadvantages, namely it reduces the molar absorpticity of azo pigments and reacts with the diazo derivative to give a yellow chromophore. Furthermore, all these methods have several deficiencies, namely feasibility of automation, complex nature of accelerator and of alkaline reagents, and above all sensitivity to hemoglobin interference, which is inter alias a common contaminant of pediatric serum samples.

The use of zinc sulfate in the determination of bilirubin is known from EP-A-0 433 977, as is the use of dimethylformamide from WO-A-9 904 258.

The purpose of the present invention is therefore to provide new reagents for use in the method of determination of bilirubins which do not present the disadvantages and deficiencies of the known methods.

A first object of the invention is thus a reagent as accelerator for use in the determination of bilirubin in biological sera which is constituted by dimethylformamide.

A second object of the invention is a reagents composition for use in the determination of bilirubin in biological sera, which comprises dimethylformamide, zinc sulfate and 6-hydroxy-2,5,7,8-tetramethyl chroman-2-carboxylic acid.

A third object of the invention is a method for the determination of bilirubins in biological sera, which comprises mixing a serum sample with sodium nitrite and sulfanilic acid in presence of a reagents composition comprising dimethylformamide as accelerator of the diazotization reaction, zinc sulfate as chromophore stabilizer and 6-hydroxy-2,5,7,8-tetramethyl chroman-2-carboxylic acid, then adding 2-aminoethanol or a mixture of 2-aminoethanol and dimethylformamide, and finally measuring the absorbance of the mixture obtained.

The reagents composition of the present invention may be the following more particularly for use in the determination of total bilirubin : 50 ml of dimethylformamide, 25 mg of zinc sulfate and 1.5 ml of a 0.1 M solution of 6-hydroxy-2,5,7,8-tetramethyl chroman-2-carboxylic acid, with water added up to 100 ml.

For the determination of total bilirubin, the above reagents composition (7 ml) is added to a mixture of 0.075 ml of sodium nitrite (5g/lt) (0,072 mole/l ) and 1 ml of sulfanilic acid (prepared by dissolving 5 g of sulfanilic acid in water, adding 5 ml of concentrated HCl and diluting to 1lt with water); then 0.7 ml of the above mixture is mixed with 0.02 ml of serum sample, and 0.1 ml of 2-aminoethanol is added before proceeding with the measurement of the absorbance at 600 nm against water.

The reagents composition of the present invention may be the following, more particularly for use in the determination of direct bilirubin : 12 ml of dimethylformamide, 37.5 mg of zinc sulfate and 1 ml of a 0.1M solution of 6-hydroxy-2,5,7,8-tetramethyl chroman-2-carboxylic acid, with water added up to 100 ml.

For the determination of direct bilirubin, the above reagents composition (7ml) is added to a mixture of 0.075 ml of sodium nitrite and 1 ml of sulfanilic acid (as for the determination of total bilirubin); then 0.35 ml of this mixture is mixed with 0.02 ml of serum sample, and 0.45 ml of a mixture of 7 ml of dimethylformamide with 2 ml of 2-aminoethanol is added before proceeding with the measurement of the absorbance at 600 nm against water.

In the method according to the present invention, bilirubin (Bu, mBc, dBc and Bs) reacts with diazotized sulfanilic acid in the presence of the accelerator (dimethylformamide) and the stabilizer (zinc sulfate) under very mild conditions and short reaction time to give pink azopigments. The latter are transformed into stable blue derivatives by the addition of the single alkaline reagent (2-aminoethanol), the intensity of said derivatives being used for quantification.

Furthermore, the presence of the other ingredient in the reagents composition, i.e 6-hydroxy-2,5,7,8-tetramethyl chroman-2-carboxylic acid, totally eliminates interference by hemoglobin by providing a protective effect on bilirubin.

The present invention will now be described in more details by reference to the following Example, which is of course given here only as such without limiting the scope of the invention, and to the annexed drawings.

Figure 1 shows the absorption of the diazo bilirubic derivates formed.
Figure 2 shows the absorbance vs/ concentration according to the method of the invention and that of Doumas.
Figure 3 is representing the standard curve of molar absorpitvity of bilirubin assay under reaction conditions of the present method.

Figure 4 illustrates the effect of Trolox on interference of hemoglobin on apparent bilirubin values.

Figure 5 shows the respective expected values of total bilirubin of several samples at different concentrations.

Figure 6 shows the absorbance vs/ concentration according to the method of the invention and that of Jendrassik.

Figure 7 illustrates the effect of Trolox on interference of hemoglobin on bilirubin concentration.

Figure 8 shows the respective expected values of direct bilirubin of several samples at different concentrations.

Figure 9 shows the linearity of the method.

In this example, the following equipments, materials and type of reagents have been used:

- Instruments : UV 160 U Model, UV-Visible recording spectrophotometer (Shimadzu, JP) and Suprasil (QS) blackwall Beckmann Cell 900 (Beckman Instruments, US); for the automated mode, Hitachi 704 automatic analyser (Boehringer Mannheim, DE).
- Materials : bilirubins and ditaurobilirubins from Calbiochem (La Jolla, CA, US).
- Reagents and standards : analytical grade reagents from Fluka (Buchs, CH); sera from normals (unjaundiced, unhemolyzed) and from patients with various disorders of bilirubin metabolism (separated immediately after venipuncture and stored at -45°C).

(1) Reagents for total bilirubin :

- Sodium nitrite : 5.0 g/lt prepared freshly every month and kept at 4°C.
- Sulfanilic acid : 5.0 g/lt prepared by dissolving sulfanilic acid in water, adding 5.0 ml of concentrated HCl and diluting to 1 lt with water; stored at room temperature.
- Composition of the invention : 25.0 mg of 6-hydroxy-2,5,7,8-tetramethyl chroman-2-carboxylic acid and 1.5 ml of 0.1 M solution of zinc sulfate are added to 50.0 ml of dimethylformamide, and the volume is made up to 100.0 ml with deionized water; the composition can be stored at + 4°C and should be discarded when discolored.
- R1 reagent: prepared by mixing 0.075 ml of sodium nitrite, 1.0 ml of sulfanilic acid, followed by the addition of 7.0 ml of composition of the invention. R1 can be used within 24 hr at room temperature or 120 hr at + 4°C.
- R2 : alkaline reagent constituted by 2-aminoethanol.

(2) Reagent for direct bilirubin

- Composition of the invention : 37.5 mg of 6 hydroxy-2,5,7,8-tetramethyl chroman-2-carboxylic acid and 1.0 ml of 0.1 M solution of zinc sulfate are added to 12.0 ml of dimethylformamide, and the volume is made up to 100.0 ml with deionized water; the composition can be stored at + 4°C and should be discarded when discolored.
- R1 reagent: see (1), but with above composition of the invention.
- R2 : alkaline reagent obtained by mixing 7.0 ml of dimethylformamide with 2.0 ml of 2-aminoethanol.

(3) Standard bilirubin solutions : these are prepared by dissolving in a small amount of dimethyl sulfoxide and 0.1 M Na2Co3 followed by dilution in 40 g/lt of albumin in 0.1 mol/lt of Tris buffer pH 7.4 or in clear, unjaundiced, unhemolyzed human serum.

(4) Testing procedures for non-hemolyzed samples :

A. For total bilirubin :
In a small test tube, mix 0.020 ml of serum sample with 0.700 ml of R1. After two minutes, add 0.1 ml of R2 and mix. Measure absorbance at 600 nm against water. A sample of appropriate standard is treated similarly and used for calculation.

B. For direct bilirubin :
In a small test tube, mix 0.02 ml of serum sample with 0.350 ml of R1. After five minutes, add 0.450 ml of R2 and mix. Measure absorbance at 600 nm against water. A sample of appropriate ditaurobilirubin standard is treated similarly and used for calculation.

(5) Testing procedures for hemolyzed samples:

- The volumes of sample and reagents are the same as above, but use the equations stated under results for calculation.

(6) Testing procedures using Hitachi 704 automatic analyzer. Volumes of sample, R1 and R2 are reduced by 50% of their values mentioned in the manual mode mentioned above.

Results

I. Manual mode for total bilirubin.

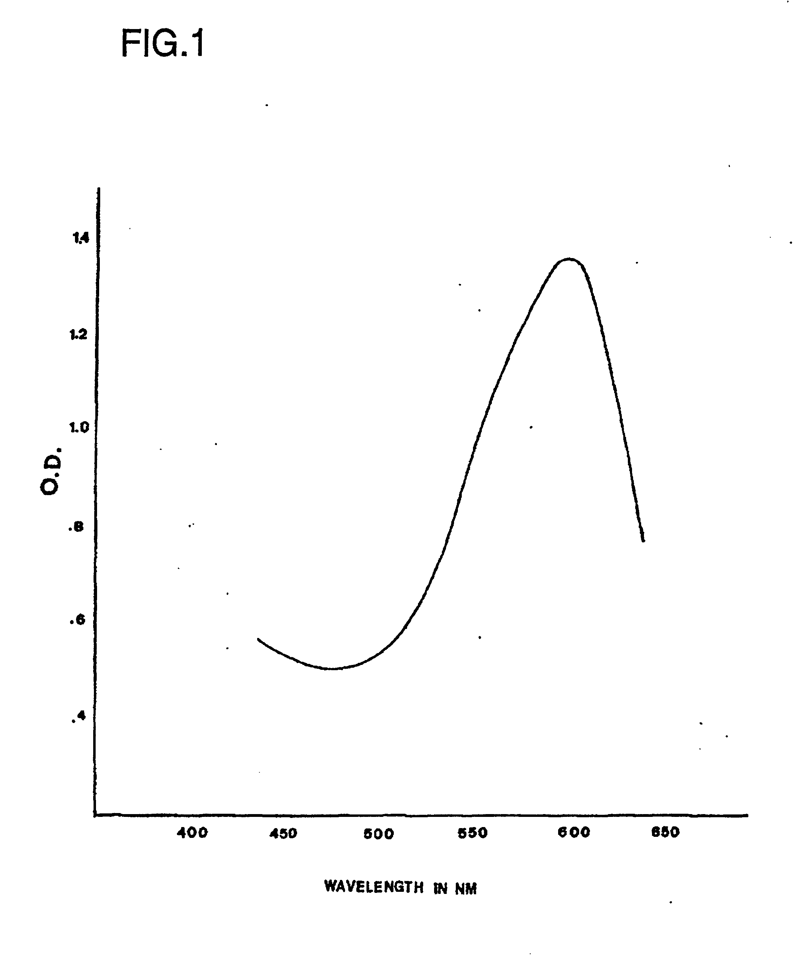

Figure 1 shows the absorption of diazo bilirubic derivates formed under the experimental conditions of the method according to the present invention, which have a chromophore absorbing maximally at 600 nm.

It has been confirmed that the diazo derivative of sulfamilic acid produces the violet /pink diazo pigment of bilirubin, and that said pigment is transformed into a blue chromophore by the addition of the reagent 2-aminoethanol.

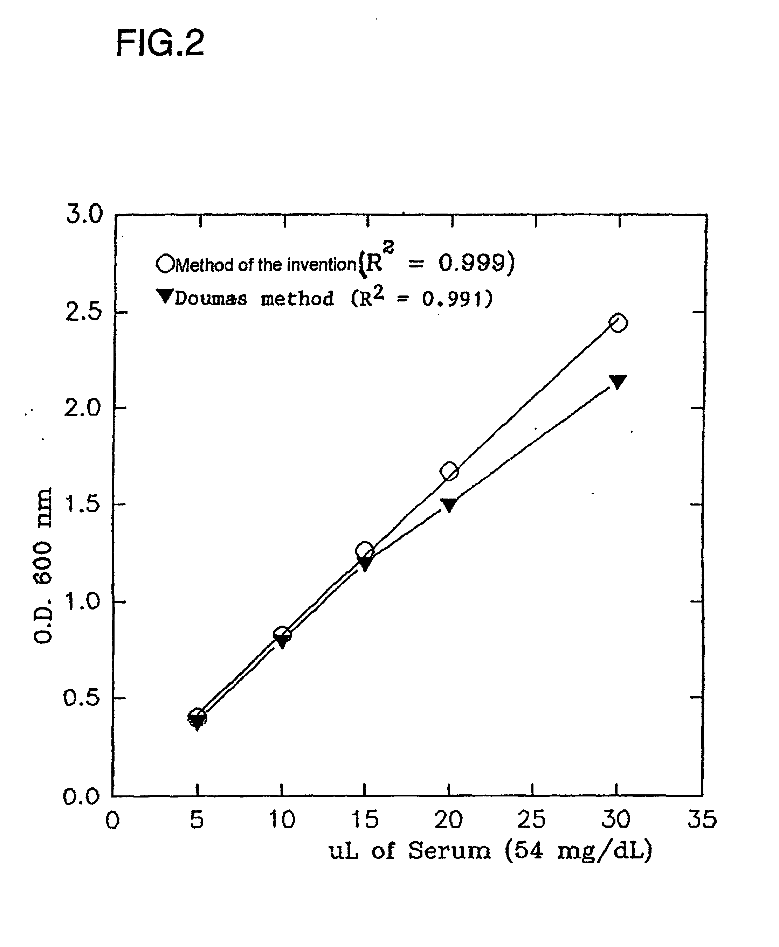

Figure 2 confirms that the relation between absorbance and concentration is linear. On comparison with Doumas method, the present method assumes perfect linearity up to an absobance of 2.5A, where Doumas method failed to be linear at lower values of absorbance. Furthermore, Pearson's coefficient (r2) is almost 1.0.

The within-run precision calculated from samples (n=20) using Dade® L-Sta-Bil Lot No. BIC-983K containing about 20 mg of bilirubin/dl is excellent with a C.V. of 1,95% and S.D. of 4,72 g x $10^{-3.}$ Another group of samples (n=18) containing about 5 mg/dl showed a C.V. of 2.74% and S.D. of 2.77 x $10^{-3}$. Between-day precision for samples preserved at -45°C (n=12) showed a C.V. of 4.48% and S.D. of $1x10^{-2}$.

The colour produced as a result of the reaction of the invention is very stable whether at very high O.D. of 1.4 or at low O.D. of 0.1. The d A/min ranged from 0.0 to 0.0001 over a period of 20 minutes.

The lower limit of detection of this method based on 2xS.D. of the reading of the low control is 0.2 mg/dl.

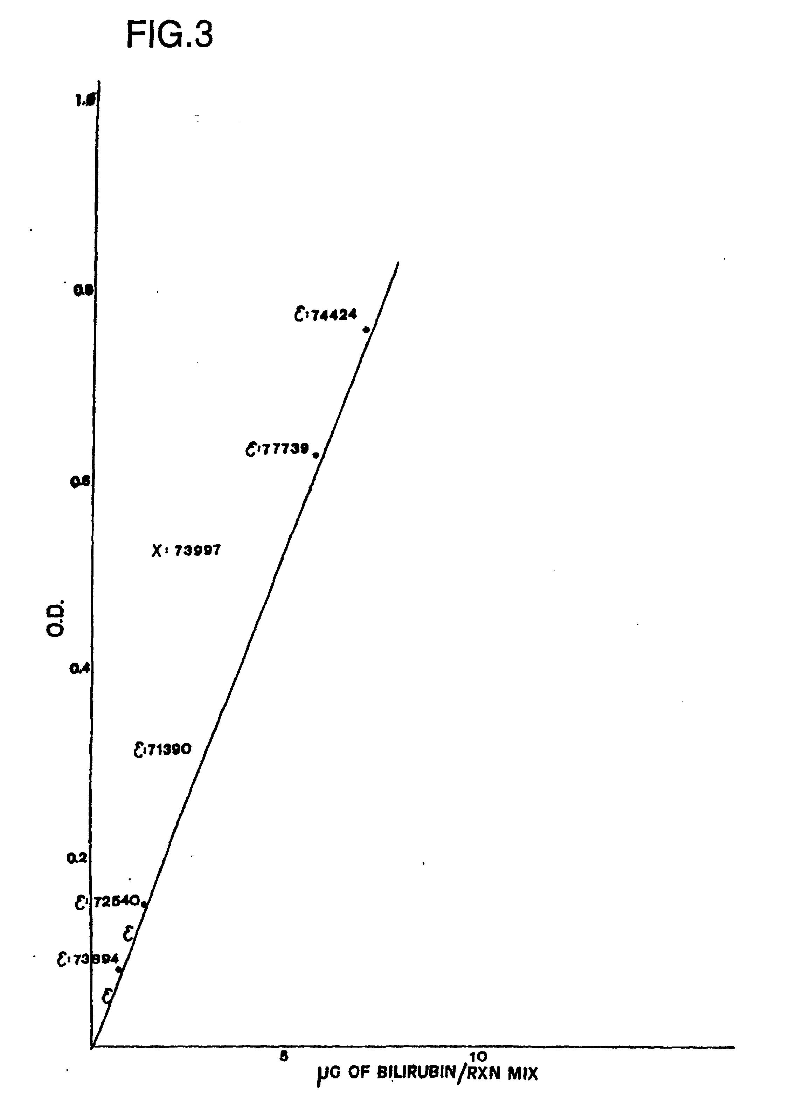

Figure 3 represents the standard curve of molar absorptivity of bilirubin assay under reaction conditions of the present method, which is more sensitive than that of Doumas by about 20% and this extends to the calculated value of the molar absorptivity (E) of azo bilirubin.

It has further been found that the ratio of serum to reagent R1 that does not cause protein precipitation was of 1:15. Even at a higher ratio of 1:7.5 where some precipitation take place, the addition of the alkaline reagent 2-aminoethanol promotes dissolution.

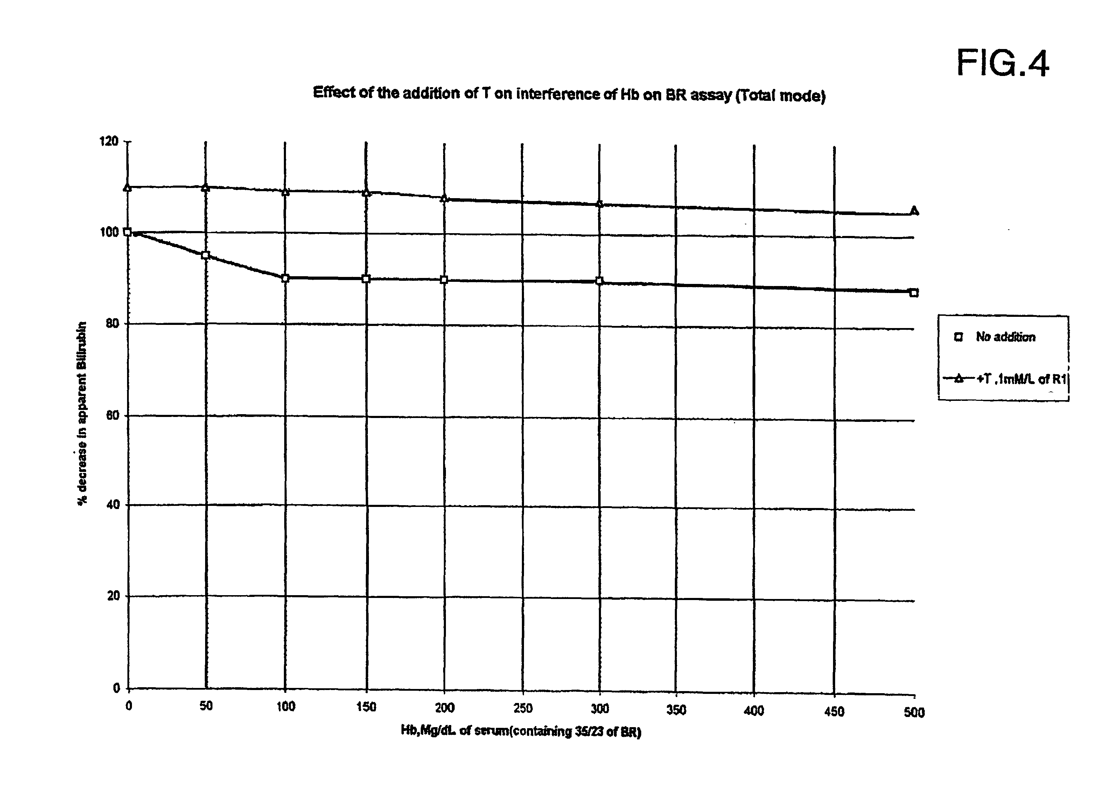

As already mentioned, hemoglobin has an interference effect on the measures, and apparent bilirubin values decrease with increasing hemoglobin concentrations. Figure 4 shows this effect, as well as the protective effect of Trolox CR (i.e. 6-hydroxy-2,5,7,8-tetramethyl chroman-2-carboxylic acid), which in fact totally remove the hemoglobin negative effect.

However, as bilirubin concentration falls, an increase in apparent bilirubin is observed with increasing hemoglobin. This is explained by the fact that hemoglobin has an absorbance at about 536 nm which extends to the region of maximum alkaline diazo bilirubin at 600 nm. Therefore, hemoglobin even in the presence of Trolox CR may have a positive interference effect. This property can be used to calculate bilirubin concentration at any hemoglobin values.

Calculation of bilirubin concentration using the spectrophotometric principle of multicomponent analysis:

Since the spectra of hemoglobin and alkaline diazo bilirubin overlap, the following two equations can be used to calculate bilirubin concentration :

$$A1^{600} = a1^{Hb} c^{Hb} + a1^{BR} c^{BR}$$

$$A2^{536} = a2^{Hb} c^{Hb} + a2^{BR} c^{BR}$$

By running many samples of increasing concentation of hemoglobin, bilirubin under the present assay conditions, averaging the values of molar absorptivities of each, the concentration of total bilirubin in a sample in the presence of any hemoglobin concentration is found using the following equation :

$$C^{TBR} = 2.43 \, A1 - A2 \times 13.84 \text{ in mg/dl}$$

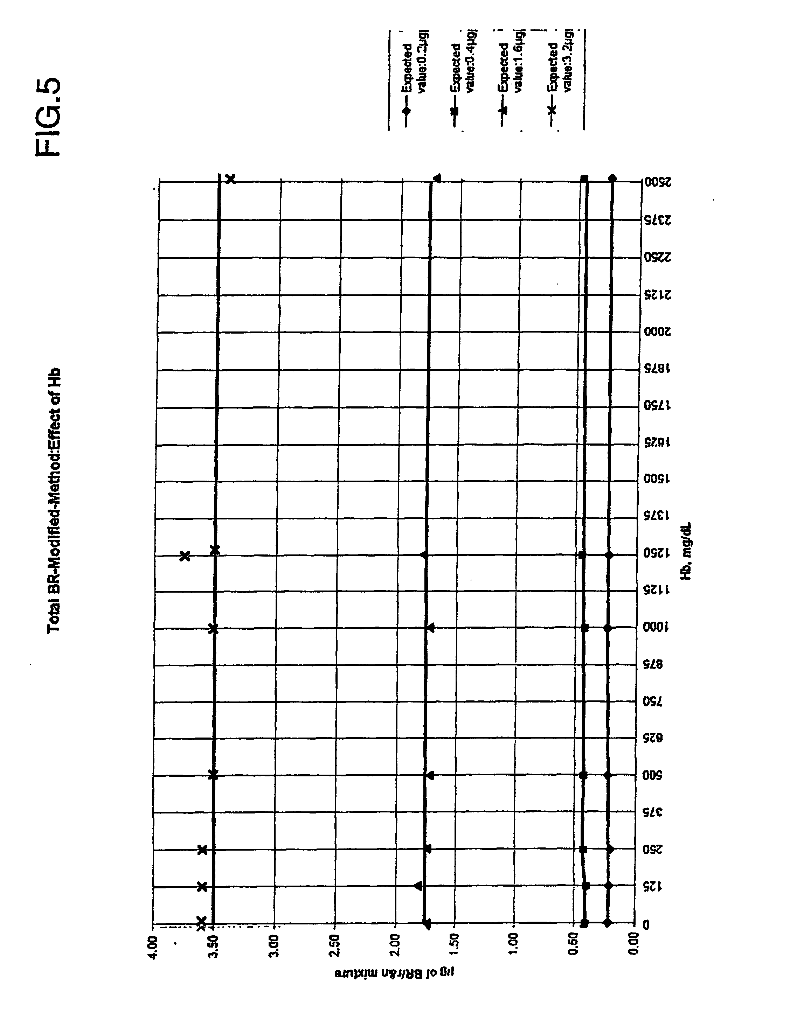

As shown in figure 5, the expected values of total bilirubin in many samples at very different concentrations remain constant even the presence of 2500 mg/dl of hemoglobin. The within-run precision of this method for samples containing a total bilirubin up to 5.0 mg/dl is excellent. Similar results were obtained whether Hb was of adult or foetal origin. The procedure was shown to be linear up to about 55 mg of bilirubin in 100 ml of serum.

II. Manual mode for direct bilirubin.

In case of direct bilirubin, the reaction is complete after 5 min.

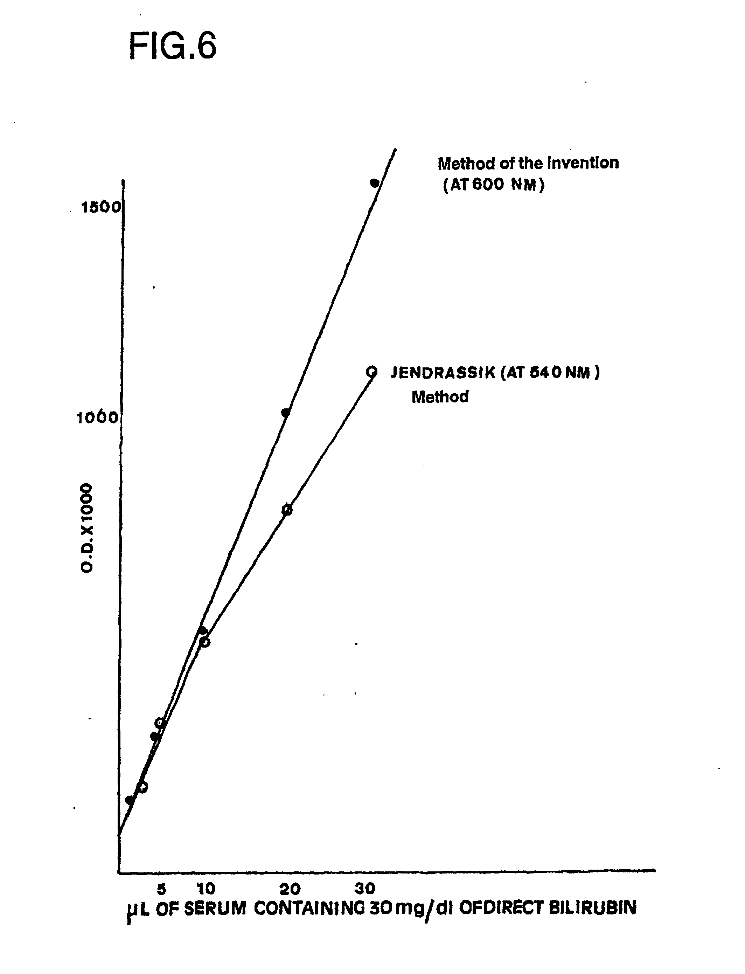

Figure 6 shows that the method of the present invention is superior to that of Jendrawik adopted to Hitachi 911. It is perfectly linear and more sensitive at high concentration by about 50%.

The serum ratio to R1 ratio can be increased up to 1:35 without salting out of proteins.

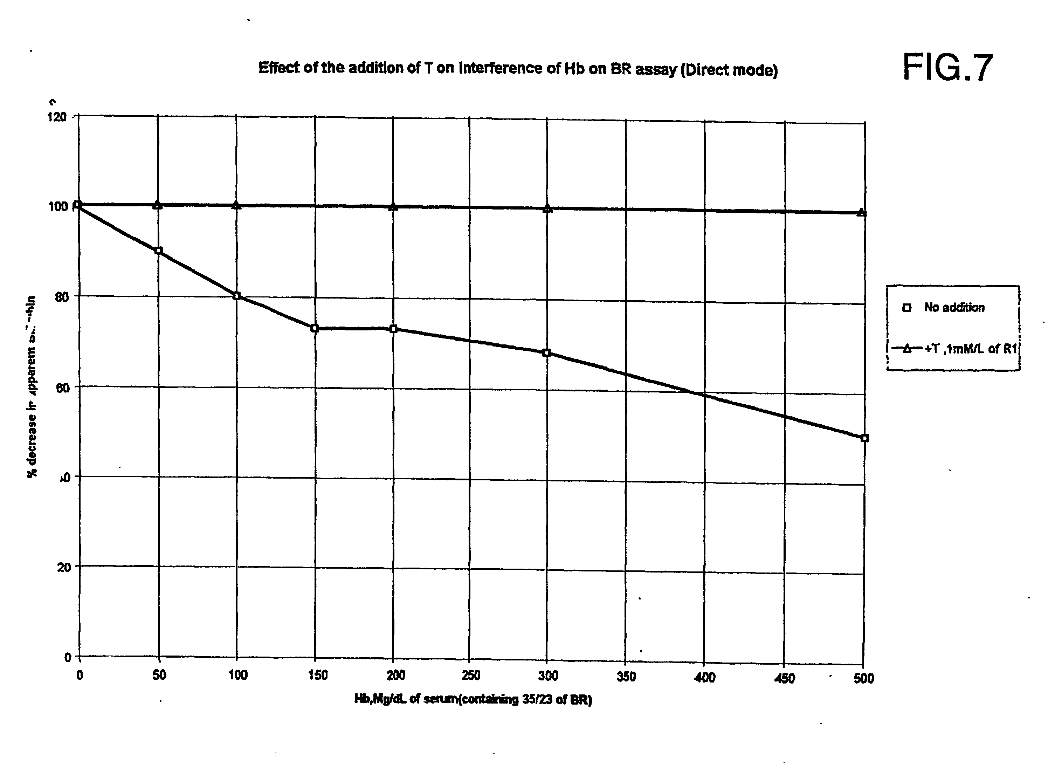

Figure 7 shows that the presence of about 500 mg/dl of hemoglobin in serum decreases the bilirubin concentration by about 50%, but that this negative effect is completely avoided by the addition of Trolox CR which thus provides an efficient protective effect

Ditaurobilirubin and hemoglobin prepared freshly from R.B.C. hemolyzates were used to establish the equation appropriate for the calculation of direct bilirubin concentration. The method followed was the same as for total bilirubin mentioned before. The equation resulting from such treatment was:

$$C^{DBR} = 2.615 \text{ A1} - \text{A2} \times 19.0, \text{ mg/dl as DTB}$$

$$C^{DBR} = 2.615 \text{ A1} - \text{A2} \times 13.0, \text{ mg/dl as BR}$$

This equation is modified to account for the interference of unconjugated bilirubin that reacts in the direct mode as follows:

$$C^{DBR} = 2.615 \text{ A1} - \text{A2} \times 9.0, \text{ mg/dl as DBR}$$

The procedure is linear up to 30 mg of direct bilirubin in 100 ml of serum, and furthermore has excellent within-sample precision.

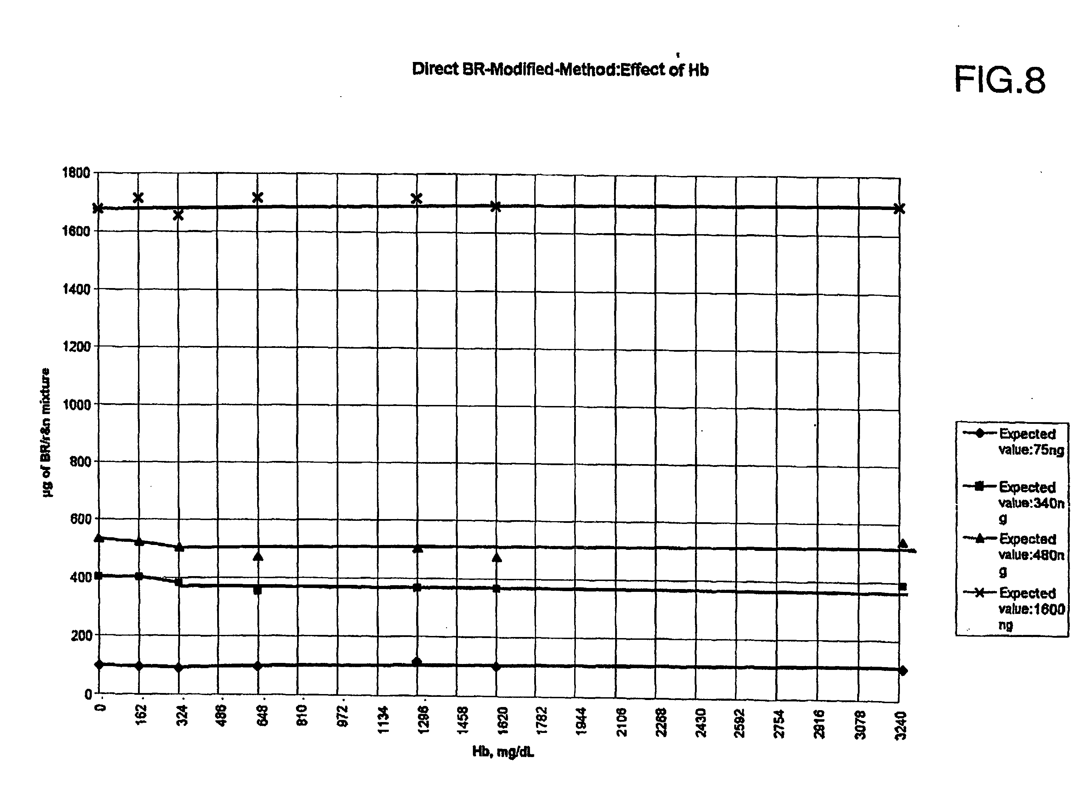

As shown in Figure 8, the expected values of direct bilirubin in many samples remain constant even in the presence of more than 3000 mg of hemoglobin in 100 ml of serum.

III. Automation

- Bichromatic analysis for total bilirubin

As mentioned previously and because of hemoglobin interference, the method of the invention requires measurements at two wavelengths, namely 600 and 536 nm. However, on the Hitachi 704, only 600 and 546 nm are available, and therefore the samples were read first at 600 nm and then at 546 nm.

At each time, a secondary wavelength at 700 nm was used to eliminate interference by serum components.

For calculation of concentration, two solutions of bilirubin were prepared with and without hemoglobin and read on Monitor Data, two numbers, one reflecting absorbance at 600 nm and the other at 546 nm. From these numbers, it was possible to generate two equations with two unknowns and when the equations were solved, resulting to an equation relating absorbance at two wavelengths, i.e. 4.95 B1 (at 600 nm)- 2.5. B2 (at 546). This equation was entered into the computer which gives the concentration of bilirubin directly in mg/dl.

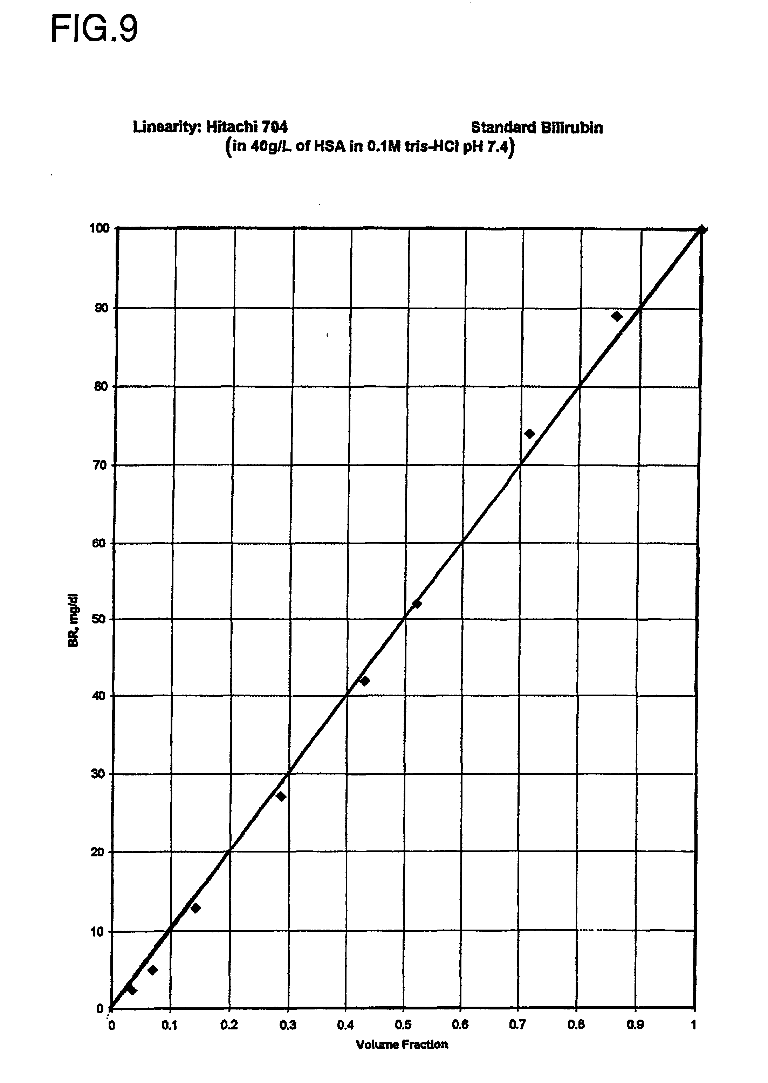

The method was found to be linear up to at least 100 mg/dl, as shown in Figure 9. At the same time, it is highly sensitive and a value so low as about of 0.05 mg/dl can be easily calculated.

- Bichromatic analysis for direct bilirubin :

- As above, a solution of ditaurobilirubin in HSA with and without HB was used to generate two equations with two unknowns. The resulting equation 3.97 B1 -1.67 B2 was used to check the linearity and sensitivity of the method . As a result, it was shown that it is linear up to at least 60 mg/dl and sensitive to about 0.05 mg/dl.
- Bichromatic analysis for direct bilirubin in human sera:
    The presence of unconjugated bilirubin in human sera falsely increases the direct bilirubin. Therefore, a different equation is derived that applies in the presence of both direct and indirect bilirubins as follows:

    a) DTB was added to pooled serum at a final concentration of 3.45 mg/dl in BR equivalent;
    b) to another sample, 0,5 ml of Hb solution of 4.0 g/dl was added.

Both samples were run (x20) on Hitachi 704 in the direct BR mode and BR content was calculated using the formula for total BR, i.e 4.95 B1-2.5 B2.

The two equations that resulted were:

$$1.062\ x - 0.717\ y = 3.79$$

$$1.63\ x - 2.43\ y = 2.25$$

on solving for y and x, the following equation was obtained: 5.38B1 - 2.68B2.

A second sample of pooled serum was run containing the same amount of Br, but as Bu and its concentration by the above formula was 2.1 mg/dl and the interference of Bu in the direct mode is 2.1/3.79 = 0.554, 1-0.554 = 0.446 is the contribution of DBR.

Therefore, the original equation should be multiplied by 0.446 to obtain another equation that reflects direct BR, which is:

$$(5.38\ B1 - 2.68\ B2) \times 0.446 = 2.40\ B1 - 1.2\ B2$$

Regarding hemoglobin interference, it has been shown that dilution of serum sample with either HSA solution or hemoglobin solution has no effect on apparent total bilirubin concentration up to at least 2285 mg of Hb in 100 ml of serum.

In the case of direct bilirubin, no hemoglobin interference is observed when BR is above 1,0 mg/dl up to about 2000 mg of Hb in 100 ml of serum. However, when direct bilirubin concentration is about 0.2 mg/dl, a hemoglobin concentration above 800 mg/dl causes severe reduction in apparent Br concentration.

No carryover between samples was observed when moving from low values (0.8 mg/l) to very high values (23.3 mg/dl) or vice-versa.

- Accuracy:

This was done by adding known increasing amounts to pooled serum specimens. Each concentration was done three times and the values were averaged. The concentrations of Bu and DTB used ranged from 1.0 mg to 25 mg/dl. Recovery was calculated using the following formula:

$$\frac{\text{Amount Found-Amount Originally Present} \times 100}{\text{Amount added}}$$

The recovery in both cases ranged from 99 to 108 % with an average of 102 %.

- Values in humans according to Hitachi 704 (method of the invention). One hundred samples obtained from ostensibly adult healthy persons were analyzed. The results were as follows:

| Bilirubin species | Range, mg/dl | Mean | S.D. |
|---|---|---|---|
| TBR | 0.097-0.980 | 0.50 | 0.23 |
| DBR | 0.036-0.384 | 0.21 | 0.10 |

The various features of the method according to the present invention have been reported, in addition to other features that were not detailed above, on the following Table.

TABLE

| Comparison of Features of TBR and DBR using Hitachi 704 | | | |
|---|---|---|---|
| Feature | | TBR | DBR |
| 1. | Standards | Unconjugated BR (Calibiochem) | Ditaurobilirubin(Calibiochem) |
| 2. | Diazotime,S: | 300 | 300 |
| 3. | Wavelength , nm | 600/700& 546/700 | 600/700 &546/700 |

TABLE   (continued)

| Feature | | TBR | DBR |
|---|---|---|---|
| | | Comparison of Features of TBR and DBR using Hitachi 704 | |
| 4. | Bichromatic: | Yes | Yes |
| 5. | Specimen blank: | None | None |
| 6. | Turbidity effect[a] | Increase of 0.048 mg/dl at TG of 650 mg/dl | Increase of 0.02mg/dl at TG of 650 mg/dl |
| 7. | Hgb interference[b]: | 800 at 0.2<br>2200 at 1.2<br>4000 at 5.0 | As TBR |
| 8. | Effect of anticoagulant[c]: | None | None |
| 9. | Matrix effects: | None in total mode observed in the direct mode | None in either mode |
| 10. | Linear to , mg/dl (Umol/L) | 100 (1710) | 60(1026)[d] |
| 11. | Lower limit of detection[e] mg/dl , (Umol/L) | 0.05(0.85) | 0.05(0.85) |
| 12. | Accuracy, recovery,% (concentration range, 1-25mg/dl) | 102 | 102 |
| 13. | Precision[f] within samples(21)[g] | 0.64, 0.02, 2.92<br>5.48, 0.06, 1.09 | 0.33, 0.01, 3.79<br>1.38, 0.02, 1.61 |
| 14. | Carry over | None | None |
| 15. | Requirements for serum-matrix standards | None | None |
| 16. | pH of reaction mixture | 2.45 | 2.45 |
| 17. | Vol. Fraction, final | 0.025 | 0.025 |
| 18. | HCL, final concentration (mmol/l) in R1 | 7.5 | 7.5 |
| 20. | Stability of reagents following formulation | 120h at 4°C for samples of 32mg/dl | 120h at 4°C for samples of 18mg/dl |
| 21. | Turnaround time, min | 18 | 18 |
| 22. | Correlation with reference or preferred method | Excellent with Doumas method | Excellent with Michaelson-Gambino |
| 23. | Normal range in adults | <1.0 mg/dl | method <0.4 mg/dl |
| 24. | Cost, $ | 0.005 | <0.005 |

a: TG: Triglycerides

b: concentration of Hgb in mg/dl at BR concentration in mg/dl at which interference is observed

c:Heparin, Citrate , EDTA

e: LLD=XL+KSB1 where X=mean of sample blanks, K=3, and SB1=is S.D. of blanks.

f: Precision: Mcan, S.D., C.V. mean, S.D., C.V.

g: Between -day precision is slightly higher than the within -day precision.

**Claims**

1.  Reagents composition for the determination of bilirubins in biological sera, which comprises dimethylformamide, zinc sulfate and 6-hydroxy-2,5,7,8-tetramethyl chroman-2-carboxylic acid.

2.  Reagents composition according to claim 1, for the determination of total bilirubin, which comprises 50 ml of dimethylformamide, 25 mg of zinc sulfate and 1.5 ml of a 0.1 M solution of 6-hydroxy-2,5,7,8-tetramethyl chroman-2 carboxylic acid, with water added up to 100 ml

3.  Reagents composition according to claim 1 for determination of direct bilirubin, which comprises 12 ml of dimethylformamide, 37.5 mg of zinc sulfate and 1 ml of a 0.1 M solution of 6-hydroxy-2,5,7,8-tetramethyl chroman-2 carboxylic acid, with water added up to 100 ml.

4. Method for the determination of bilirubins in biological sera, which comprises mixing a serum sample with sodium nitrite and sulfanilic acid in presence of a reagents composition comprising dimethylformamide as accelerator of the diazotization reaction, zinc sulfate as chromophore stabilizer and 6-hydroxy-2,5,7,8-tetramethyl chroman-2-carboxylic acid, then adding 2-aminoethanol or a mixture of 2-aminoethanol and dimethylformamide, and finally measuring the absorbance of the mixture obtained.

5. Method according to claim 4 for the determination of total bilirubin, in which 7 ml of the reagents composition of claim 2 is added to a mixture of 0.075 ml of sodium nitrite (5g/lt) (0,072 mole/l) and 1 ml of sulfanilic acid (prepared by dissolving 5g of sulfanilic acid in water, adding 5 ml of concentrated HCl and diluting to 1lt with water); then, 0.7 ml of the above mixture is mixed with 0.02 ml of serum sample, and 0,1 ml of 2-aminoethanol is added before proceeding with the measurement of the absorbance at 600 nm against water.

6. Method according to claim 4 for the determination of direct bilirubin, in which 7 ml of the reagents composition of claim 3 is added to a mixture of 0.075 ml of (5g/lt) (0,07 2 mole/l) sodium nitrite and 1 ml of sulfanilic acid (prepared by dissolving 5 g of sulfanilic acid in water, adding 5 ml of concentrated HCl and diluting to lit with water); then, 0.35 ml of this mixture is mixed with 0.02 ml of serum sample, and 0.45ml of a mixture of 7 ml of dimethylformamide with 2 ml of 2-aminoethanol is added before proceeding with the measurement of the absorbance at 600 nm against water.

**Patentansprüche**

1. Reagenzienzusammensetzung für die Bestimmung von Bilirubinen in biologischen Sera, welche Dimethylformamid, Zinksulfat und 6-Hydroxy-2,5,7,8-tetramethylchroman-2-carbonsäure umfasst.

2. Reagenzienzusammensetzung gemäß Anspruch 1, zur Bestimmung von Gesamt-Bilirubin, welche 50 ml Dimethylformamid, 25 mg Zinksulfat und 1,5 ml einer 0,1 M Lösung von 6-Hydroxy-2,5,7,8-tetramethylchroman-2-carbonsäure, mit Wasser bis auf 100 ml aufgefüllt, umfasst.

3. Reagenzienzusammensetzung gemäß Anspruch 1 zur Bestimmung von direktem Bilirubin, welche 12 ml Dimethylformamid, 37,5 mg Zinksulfat und 1 ml einer 0,1 M Lösung von 6-Hydroxy-2,5,7,8-tetramethylchroman-2-carbonsäure, mit Wasser bis auf 100 ml aufgefüllt, umfasst.

4. Verfahren zur Bestimmung von Bilirubinen in biologischen Sera, welches das Mischen einer Serum-Probe mit Natriumnitrit und Sulfanilsäure in Anwesenheit einer Reagenzienzusammensetzung, die Dimethylformamid als Beschleuniger für die Diazotierungsreaktion, Zinksulfat als Chromophor-Stabilisator und 6-Hydroxy-2,5,7,8-tetramethylchroman-2-carbonsäure umfasst und dann die Zugabe von 2-Aminoethanol oder einer Mischung von 2-Aminoethanol und Dimethylformamid und schließlich die Messung der Extinktion der erhaltenen Mischung, umfasst.

5. Verfahren gemäß Anspruch 4, zur Bestimmung von Gesamt-Bilirubin, in welchem 7 ml der Reagenzienzusammensetzung von Anspruch 2 zu einer Mischung aus 0,075 ml Natriumnitrit (5 g/l) (0,072 mol/l) und 1 ml Sulfanilsäure (hergestellt durch Lösen von 5 g Sulfanilsäure in Wasser, Hinzugeben von 5 ml konzentrierter HCl und Verdünnung mit Wasser auf 1 l) hinzugegeben werden; dann werden 0,7 ml der obigen Mischung mit 0,02 ml Serum-Probe gemischt und 0,1 ml 2-Aminoethanol hinzugefügt, bevor die Messung der Extinktion bei 600 nm, gegen Wasser, durchgeführt wird.

6. Verfahren gemäß Anspruch 4 zur Bestimmung von direktem Bilirubin, in welchem 7 ml der Reagenzienzusammensetzung von Anspruch 3 zu einer Mischung aus 0,075 ml Natriumnitrit (5 g/l), (0,072 mol/l) und 1 ml Sulfanilsäure (hergestellt durch Lösen von 5 g Sulfanilsäure in Wasser, Hinzugeben von 5 ml konzentrierter HCl und Verdünnung mit Wasser auf 1 l) hinzugegeben werden; dann werden 0,35 ml dieser Mischung mit 0,02 ml Serum-Probe gemischt und 0,45 ml einer Mischung aus 7 ml Dimethylformamid und 2 ml 2-Aminoethanol hinzugegeben, bevor die Messung der Extinktion bei 600 nm, gegen Wasser, durchgeführt wird.

**Revendications**

1. Composition de réactifs pour la détermination de bilirubines dans des sérums biologiques, qui comporte du diméthylformamide, du sulfate de zinc et de l'acide 6-hydroxy-2,5,7,8-tétraméthyle chromane-2-carboxylique.

**2.** Composition de réactifs selon la revendication 1, pour la détermination de bilirubine totale, qui comporte 50 ml de diméthylformamide, 25 mg de sulfate de zinc et 1,5 ml d'une solution 0,1 M d'acide 6-hydroxy-2,5,7,8-tétraméthyle chromane-2-carboxylique, avec de l'eau ajoutée jusqu'à 100 ml.

**3.** Composition de réactifs selon la revendication 1 pour la détermination de bilirubine directe, qui comporte 12 ml de diméthylformamide, 37,5 mg de sulfate de zinc et 1 ml d'une solution 0,1 M d'acide 6-hydroxy-2,5,7,8-tétraméthyle chromane-2-carboxylique, avec de l'eau ajoutée jusqu'à 100 ml.

**4.** Procédé pour la détermination de bilirubines dans des sérums biologiques, qui comporte le mélange d'un échantillon de sérum avec du nitrite de sodium et de l'acide sulfanilique en présence d'une composition de réactifs comportant du diméthylformamide en tant qu'accélérateur de la réaction de diazotation, du sulfate de zinc en tant qu'agent stabilisant de chromophore et de l'acide 6-hydroxy-2,5,7,8-tétraméthyle chromane-2-carboxylique, l'ajout ensuite de 2-aminoéthanol ou d'un mélange de 2-aminoéthanol et de diméthylformamide, et la mesure en fin de compte de l'absorbance du mélange obtenu.

**5.** Procédé selon la revendication 4 pour la détermination de bilirubine totale, dans lequel 7 ml de la composition de réactifs selon la revendication 2 sont ajoutés à un mélange de 0,075 ml de nitrite de sodium (5 g/l) (0,072 mole/l) et de 1 ml d'acide sulfanilique (préparé en dissolvant 5 g d'acide sulfanilique dans de l'eau, en ajoutant 5 ml de HCl concentré et en diluant jusqu'à 1 l à l'aide d'eau), alors, 0,7 ml du mélange ci-dessus est mélangé avec 0,02 ml d'un échantillon de sérum, et 0,1 ml de 2-aminoéthanol est ajouté avant d'effectuer la mesure de l'absorbance à 600 nm contre de l'eau.

**6.** Procédé selon la revendication 4 pour la détermination de bilirubine directe, dans lequel 7 ml de la composition de réactifs selon la revendication 3 sont ajoutés à un mélange de 0,075 ml (5 g/l) (0,072 mole/l) de nitrite de sodium et de 1 ml d'acide sulfanilique (préparé en dissolvant 5 g d'acide sulfanilique dans de l'eau, en ajoutant 5 ml de HCl concentré et en diluant jusqu'à 1 l à l'aide d'eau), alors, 0,35 ml de ce mélange est mélangé avec 0,02 ml d'un échantillon de sérum, et 0,45 ml d'un mélange de 7 ml de diméthylformamide avec 2 ml de 2-aminoéthanol est ajouté avant d'effectuer la mesure de l'absorbance à 600 nm contre de l'eau.

## FIG.1

# FIG.2

# FIG.3

# FIG.4

Effect of the addition of T on interference of Hb on BR assay (Total mode)

Legend:
- □ No addition
- △ +T .1mM/L of R1

X-axis: Hb,Mg/dL of serum(containing 35/23 of BR)

Y-axis: % decrease in apparent Bilirubin

# FIG.5

Total BR-Modified-Method:Effect of Hb

# FIG.6

Method of the invention
(AT 600 NM)

JENDRASSIK (AT 540 NM)
Method

O.D.X1000

1500

1000

5   10      20      30

μL OF SERUM CONTAINING 30 mg/dl OFDIRECT BILIRUBIN

**Effect of the addition of T on interference of Hb on BR assay (Direct mode)**

# FIG.7

Legend:
- □ No addition
- △ +T ,1mM/L of R1

X-axis: Hb,Mg/dL of serum(containing 35/23 of BR)

Y-axis: % decrease in apparent bilirubin

FIG.8

# FIG.9

**Linearity: Hitachi 704**
(in 40g/L of HSA in 0.1M tris-HCl pH 7.4)

**Standard Bilirubin**